(19) Europäisches Patentamt
European Patent Office
Office européen des brevets

(11) EP 2 030 989 A1

(12) EUROPEAN PATENT APPLICATION
published in accordance with Art. 153(4) EPC

(43) Date of publication:
04.03.2009 Bulletin 2009/10

(21) Application number: 07767111.3

(22) Date of filing: 13.06.2007

(51) Int Cl.:
*C08F 2/38* (2006.01) *C07C 319/12* (2006.01)
*C07C 323/56* (2006.01) *C07B 61/00* (2006.01)

(86) International application number:
PCT/JP2007/061885

(87) International publication number:
WO 2007/145241 (21.12.2007 Gazette 2007/51)

(84) Designated Contracting States:
AT BE BG CH CY CZ DE DK EE ES FI FR GB GR HU IE IS IT LI LT LU LV MC MT NL PL PT RO SE SI SK TR
Designated Extension States:
AL BA HR MK RS

(30) Priority: 13.06.2006 JP 2006163940

(71) Applicant: Showa Denko K.K.
Tokyo 105-8518 (JP)

(72) Inventors:
- MIYATA, Hideo
Kawasaki-shi, Kanagawa 210-0867 (JP)
- IKEDA, Haruhiko
Kawasaki-shi, Kanagawa 210-0867 (JP)
- MUROFUSHI, Katsumi
Kawasaki-shi, Kanagawa 210-0867 (JP)
- HATTORI, Yotaro
Kawasaki-shi, Kanagawa 210-0867 (JP)
- URAKAWA, Katsuro
Kawasaki-shi, Kanagawa 210-0867 (JP)

(74) Representative: Strehl Schübel-Hopf & Partner
Maximilianstrasse 54
80538 München (DE)

# (54) POLYMERIZATION ACCELERATOR, CURABLE COMPOSITION, CURED PRODUCT AND METHOD FOR PRODUCING THIOL COMPOUND

(57) A polymerization accelerator comprises a specific thiol compound. A curable composition of excellent thermal stability contains the polymerization accelerator. A cured product is obtained from the curable composition.

The polymerization accelerator comprises a thiol compound having two or more groups represented by Formula (1) below:

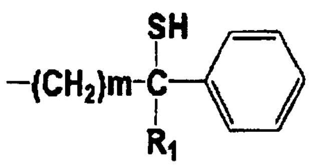

(1)

wherein $R_1$ is a hydrogen atom or a C1-10 alkyl group, and m is an integer of 0, 1 or 2.

EP 2 030 989 A1

## Description

### TECHNICAL FIELD

The present invention relates to polymerization accelerators that are used in curable compositions used as coating materials, UV or heat curable paints, molding materials, adhesives, inks, optical materials, stereolithography materials, printing plate materials and resist materials, and particularly suitable for optical materials. The invention also relates to curable compositions containing the polymerization accelerator and cured products obtained from the curable compositions. In more detail, the invention relates to polymerization accelerators comprising a specific thiol compound, curable compositions of excellent thermal stability that contain the polymerization accelerator, cured products obtained from the curable composition, and processes for producing thiol compounds.

### BACKGROUND ART

Compositions that are cured with active lights such as UV rays are used in a wide range of fields including coating materials, UV or heat curable paints, molding materials, adhesives, inks, resists, optical materials, stereolithography materials, printing plate materials, dental materials, polymer battery materials and polymer materials. For example, uses as optical materials include coating materials for optical lenses or films, cladding materials for optical fibers, and optical adhesives for optical fibers or optical lenses. Such photocurable compositions known in the art include curable compositions containing a thiol compound. These curable compositions are required to have higher performance levels with demands for higher performances in various fields such as optical materials or electronic materials. For example, improvements are required in reactivity, curing properties, optical properties of cured products such as transmittance and refractive index, adhesion to substrates, and heat resistance.

These photocurable compositions are either of one-component type or two-component type. They are cured quickly in several seconds to several minutes after radical polymerization is initiated by light irradiation between a compound having an ethylenically unsaturated double bond and a thiol compound. However, they do not satisfy stability and the curing performance at the same time. The conventional polyene/polythiol photocurable compositions have bad thermal stability; when they are stored in a liquid state, they increase viscosity and is gelled.

In detailed study of the conventional art, JP-A-2003-226718 (Patent Document 1) discloses a photocurable composition containing a specific polythiol, one or more ene compounds, and a radical photopolymerization initiator. The photocurable composition has a sulfur atom and thereby gives cured products having high refractive index and hardness.

JP-A-2003-277505 (Patent Document 2) discloses a photocurable resin composition containing a polyene, a polythiol, and a brominated aromatic compound. The composition achieves high refractive index by containing a bromine atom. The photocurable resin compositions described in JP-A-2003-226718 (Patent Document 1) and JP-A-2003-277505 (Patent Document 2) as above achieve high refractive index by containing sulfur atom, but they cannot satisfy both stability and performances such as reactivity, cure shrinkage and adhesion.

JP-A-2001-26608 (Patent Document 3) discloses a photocurable resin composition containing a polyene, a photopolymerization initiator, and not more than 50 ppm of a metal ion. By reducing metal ions, storage stability of the photocurable resin composition is obtained.

JP-A-2004-149755 (Patent Document 4) discloses a photopolymerization initiator composition that contains a mercapto group-containing thiol compound having a specific substituent and a photopolymerization initiator. This photosensitive composition has high sensitivity and good storage stability.

These compositions in the conventional art, however, are still insufficient in long-term thermal stability required in applications such as coating materials, adhesives or electronic materials.

Patent Document 1: JP-A-2003-226718
Patent Document 2: JP-A-2003-277505
Patent Document 3: JP-A-2001-26608
Patent Document 4: JP-A-2004-149755

### DISCLOSURE OF THE INVENTION

### PROBLEMS TO BE SOLVED BY THE INVENTION

It is therefore an object of the present invention to provide polymerization accelerators, curable compositions of excellent thermal stability that contain the polymerization accelerator, cured products obtained from the compositions, and processes for producing thiol compounds.

**MEANS TO SOLVE THE PROBLEMS**

The present inventors have found that the above object is achieved with curable compositions that contain polymerization initiators including a thiol compound as one component that contains two or more structures in which the carbon atom at α-position relative to the mercapto group has an aryl group in contrast to conventional primary thiol compounds. The present invention has been completed based on the finding.
That is, the present invention relates to:

[1] A polymerization accelerator comprising a thiol compound, the thiol compound having two or more groups represented by Formula (1) below:

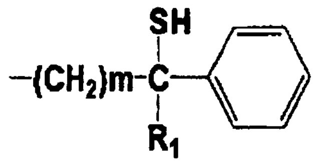

(1)

wherein $R_1$ is a hydrogen atom or a C1-10 alkyl group, and m is an integer of 0, 1 or 2.

[2] The polymerization accelerator as described in [1], wherein the thiol compound is an ester compound between a mercapto group-containing carboxylic acid represented by Formula (2) below and a polyfunctional alcohol:

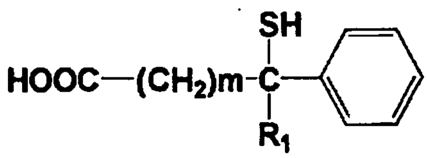

(2)

wherein $R_1$ and m are the same as $R_1$ and m in Formula (1) described in [1].

[3] The polymerization accelerator as described in [2],
wherein the polyfunctional alcohol is a compound selected from the group consisting of alkylene glycols (having a C2-10 optionally branched alkylene group), diethylene glycol, dipropylene glycol, glycerol, diglycerol, trimethylolpropane, pentaerythritol, dipentaerythritol, cyclohexanediol, cyclohexanedimethanol, norbornenedimethanol, bisphenol A, hydrogenated bisphenol A and 4,4'-(9-fluorenylidene)bis(2-phenoxyethanol).
[4] The polymerization accelerator as described in [2], wherein the thiol compound is represented by Formula (3) below:

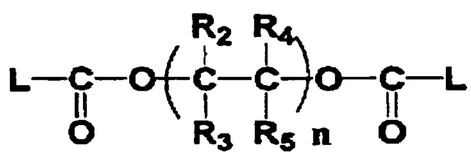

(3)

wherein $R_2$ to $R_5$ are each independently a hydrogen atom or a C1-10 alkyl group, m is an integer of 1 to 3, and L is a group represented by Formula (1) described in [1].

[5] The polymerization accelerator as described in [2], wherein the thiol compound is represented by Formula (4) below:

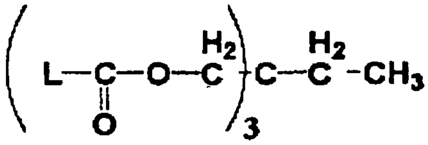

(4)

wherein L is a group represented by Formula (1) described in [1].

[6] The polymerization accelerator as described in [2], wherein the thiol compound is represented by Formula (5) below:

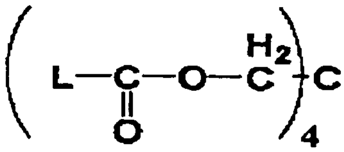

(5)

wherein L is a group represented by Formula (1) described in [1].

[7] The polymerization accelerator as described in [2], wherein the thiol compound is represented by Formula (6) below:

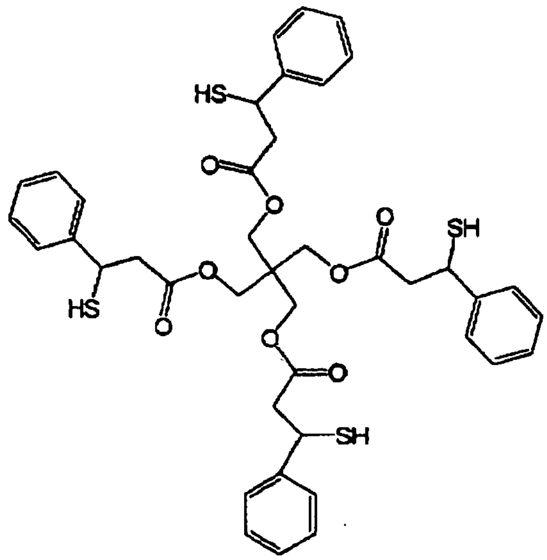

(6)

[8] The polymerization accelerator as described in [2], wherein the thiol compound is represented by Formula (7) below:

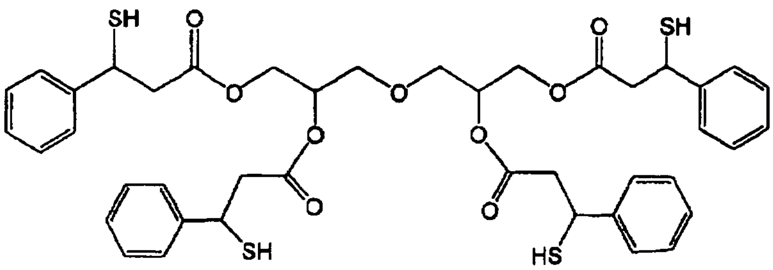

(7)

[9] A curable composition comprising the thiol compound described in any one of [1] to [8] and a radically polymerizable compound.

[10] A curable composition comprising the thiol compound described in any one of [1] to [8] and a compound having an ethylenically unsaturated double bond.

[11] A cured product obtained from the curable composition described in [9] or [10].

[12] A process for producing a thiol compound having two or more groups represented by Formula (1) described in [1], the process comprising dissolving a mercapto group-containing carboxylic acid represented by Formula (2) described in [2] and at least one compound selected from the polyfunctional alcohols described in [3] in a solvent capable of forming an azeotropic mixture with water to allow for azeotropic dehydration; adding an acid catalyst; heating the mixture under reflux; and performing azeotropic dehydration to remove water formed by the esterification.

In the above compositions, the thiol compound has structures in which the carbon atom at α-position relative to the mercapto group has an aryl group in contrast to the conventional thiol compounds in which the carbon atom at α-position relative to the mercapto group has an alkyl group irrespective of whether the thiol compounds are primary or secondary. According to having such structures the steric hindrance and electronic effect of the aryl group inhibit addition reaction of the mercapto group to an ethylenically unsaturated double bond. Consequently, the curable compositions

having improved thermal stability can be obtained.

When a polymerization initiator that is a radical generator is used simultaneously as a curable composition, the mercapto groups in the thiol compound function as radical polymerization initiating radicals for ethylenically unsaturated double bonds as soon as radical chain reaction is induced upon generation of radicals from the polymerization initiator by light or heat. In this case, the reactivity is usually reduced because of the steric hindrance around the mercapto groups in the thiol compound. According to the present invention, the reactivity is not substantially lowered because the alkyl group is changed to an aryl group. This advantage is probably because of the planar structure and electronic effect of the aryl group.

The polymerization accelerators and curable compositions obtained according to the present invention may be suitably used in a wide range of fields such as, but not limited to, coating materials, UV or heat curable paints, molding materials, adhesives, inks, optical materials, stereolithography materials, printing plate materials and resist materials.

## EFFECT OF THE INVENTION

By using as a polymerization accelerator the thiol compound having two or more groups represented by Formula (1) as described above, the steric hindrance and electronic effect of the aryl group inhibit addition reaction of the mercapto group and inhibit addition reaction of the mercapto group to an ethylenically unsaturated double bond. Consequently, the curable compositions according to the present invention achieve excellent thermal stability.

## BRIEF DESCRIPTION OF THE DRAWINGS

Fig. 1 is a $^1$H-NMR chart of 3-mercapto-3-phenylpropionic acid synthesized in Synthetic Example 1.
Fig. 2 is a $^1$H-NMR chart of pentaerythritol tetrakis(3-mercapto-3-phenylpropionate) synthesized in Synthetic Example 2.
Fig. 3 is a $^1$H-NMR chart of diglycerol tetra(3-mercapto-3-phenylpropionate) synthesized in Synthetic Example 3.

## BEST MODE FOR CARRYING OUT THE INVENTION

Embodiments of the present invention will be described in detail hereinbelow.

(Thiol compounds)

The thiol compounds used in the present invention have two or more groups represented by Formula (1). Curable compositions containing the thiol compound show improved thermal stability with controlled addition reaction to ethylenically unsaturated double bonds.

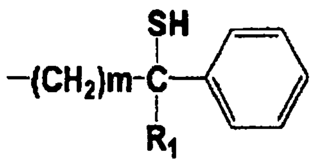

(1)

In Formula (1) above, $R_1$ is a hydrogen atom or a C1-10 alkyl group. The C1-10 alkyl groups indicated by $R_1$ may be linear or branched and include methyl, ethyl, n-propyl, iso-propyl, n-butyl, iso-butyl, tert-butyl, n-hexyl and n-octyl groups. Of these, a hydrogen atom, a methyl group and an ethyl group are preferred. The letter m is an integer of 0, 1 or 2, and is preferably 0 or 1.

The thiol compounds used in the present invention are polyfunctional thiol compounds with having two or more mercapto groups. It has been known that these polyfunctional compounds provide higher crosslinking density in radical polymerization than monofunctional compounds.

In the thiol compounds of the present invention, the mercapto group-containing groups represented by Formula (1) are preferably carboxylic acid-derived structures of Formula (8) below or ether-derived structures of Formula (9) below:

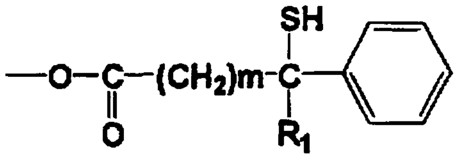

(8)

wherein $R_1$ is a hydrogen atom or a C1-10 alkyl group, and m is an integer of 0, 1 or 2.

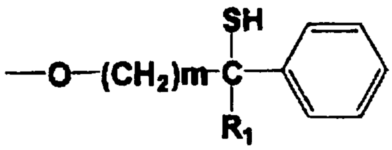

(9)

wherein $R_1$ is a hydrogen atom or a C1-10 alkyl group, and m is an integer of 0, 1 or 2.
Specific examples of the thiol compounds having structures of Formula (1) of the present invention include:

ethylene glycol bis(2-mercapto-2-phenylacetate), propylene glycol bis(2-mercapto-2-phenylacetate), diethylene glycol bis(2-mercapto-2-phenylacetate), butanediol bis(2-mercapto-2-phenylacetate), octanediol bis(2-mercapto-2-phenylacetate), cyclohexanedimethanol bis(2-mercapto-2-phenylacetate), trimethylolpropane tris(2-mercapto-2-phenylacetate), pentaerythritol tetrakis(2-mercapto-2-phenylacetate), dipentaerythritol hexakis(2-mercapto-2-phenylacetate), glycerol tris(2-mercapto-2-phenylacetate), diglycerol tetrakis(2-mercapto-2-phenylacetate), ethylene glycol bis(3-mercapto-3-phenylpropionate), propylene glycol bis(3-mercapto-3-phenylpropionate), diethylene glycol bis(3-mercapto-3-phenylpropionate), butanediol bis(3-mercapto-3-phenylpropionate), octanediol bis(3-mercapto-3-phenylpropionate), cyclohexanedimethanol bis(3-mercapto-3-phenylpropionate), trimethylolpropane tris(3-mercapto-3-phenylpropionate), pentaerythritol tetrakis(3-mercapto-3-phenylpropionate), dipentaerythritol hexakis(3-mercapto-3-phenylpropionate), glycerol tris(3-mercapto-3-phenylpropionate), diglycerol tetrakis(3-mercapto-3-phenylpropionate), ethylene glycol bis(4-mercapto-4-phenylbutyrate), propylene glycol bis(4-mercapto-4-phenylbutyrate), diethylene glycol bis(4-mercapto-4-phenylbutyrate), butanediol bis(4-mercapto-4-phenylbutyrate), octanediol bis(4-mercapto-4-phenylbutyrate), cyclohexanedimethanol bis(4-mercapto-4-phenylbutyrate), trimethylolpropane tris(4-mercapto-4-phenylbutyrate), pentaerythritol tetrakis(4-mercapto-4-phenylbutyrate), dipentaerythritol hexakis(4-mercapto-4-phenylbutyrate), glycerol tris(4-mercapto-4-phenylbutyrate), diglycerol tetrakis(4-mercapto-4-phenylbutyrate), hydrogenated bisphenol A bis(2-mercapto-2-phenylacetate), hydrogenated bisphenol A bis(3-mercapto-3-phenylpropionate), hydrogenated bisphenol A bis(4-mercapto-4-phenylbutyrate), bisphenol A dihydroxyethyl ether bis(2-mercapto-2-phenylacetate), bisphenol A dihydroxyethyl ether bis(3-mercapto-3-phenylpropionate), bisphenol A dihydroxyethyl ether bis(4-mercapto-4-phenylbutyrate), 4,4'-(9-fluorenylidene)bis(2-phenoxyethyl(2-mercapto-2-phen ylacetate)), 4,4'-(9-fluorenylidene)bis(2-phenoxyethyl(3-mercapto-3-phen ylpropionate)) and 4,4'-(9-fluorenylidene)bis(2-phenoxyethyl(4-mercapto-4-phen ylbutyrate).

In addition, examples of the thiol compounds having ether-derived structures of Formula (9) include, but are not limited to, compounds having 2-mercapto-2-phenyl methyl ether group, 2-mercapto-2-phenyl ethyl ether group or 3-mercapto-3-phenyl propyl ether group.

Preferred examples of the thiol compounds include compounds represented by Formulae (3), (4) and (5) below:

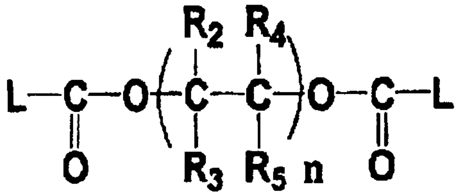

(3)

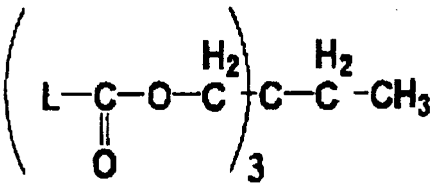

(4)

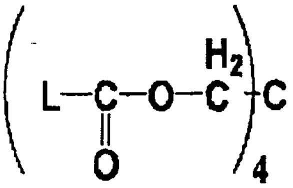

(5)

In Formula (3), $R_3$ to $R_6$ are each independently a hydrogen atom or a C1-10 alkyl group. The alkyl groups are preferably linear or branched alkyl groups having 1 to 3 carbon atoms.
Specific examples include methyl, ethyl, n-propyl and iso-propyl groups, with methyl and ethyl groups being preferred. The letter L is a mercapto-containing group represented by Formula (1).

The thiol compounds represented by Formula (3) are obtained from a diol having a C2 alkylene main chain as the polyfunctional alcohol and have two mercapto-containing groups. The thiol compounds represented by Formula (4) are obtained from trimethylolpropane as the polyfunctional alcohol and have three mercapto-containing groups. The thiol compounds represented by Formula (5) are obtained from pentaerythritol as the polyfunctional alcohol and have four mercapto-containing groups.

Preferred examples of the polyfunctional thiol compounds represented by above Formulae (3), (4) and (5) include compounds represented by Formulae (10) to (15) below:

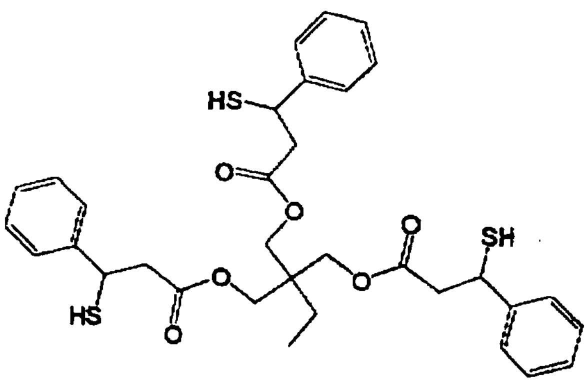

(10)

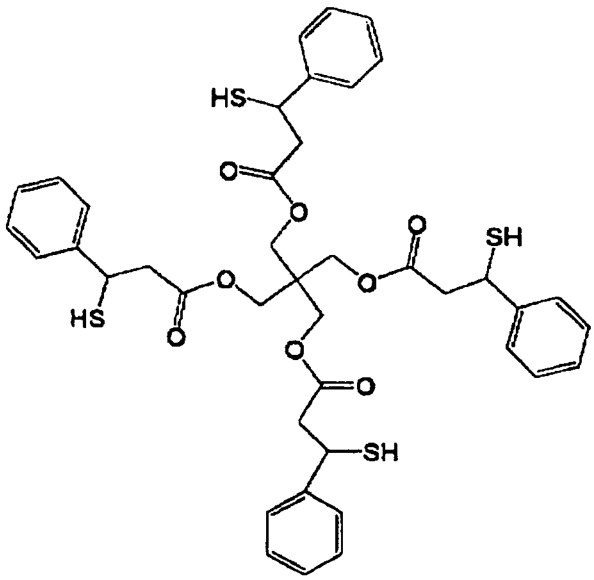

(1 1)

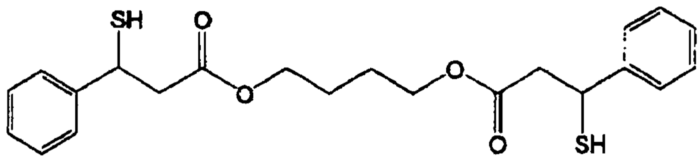

(1 2)

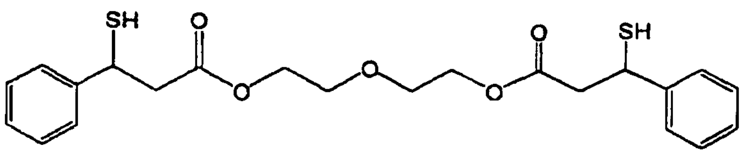

(1 3)

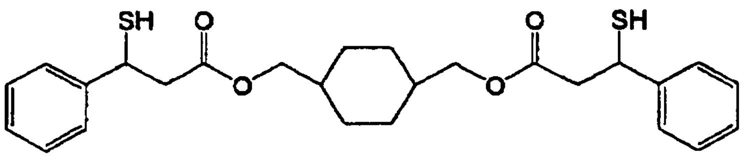

(1 4)

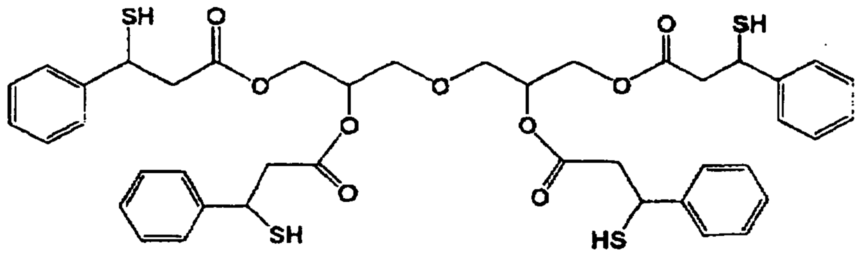

(1 5)

The molecular weight of the thiol compounds of the present invention is not particularly limited, but is preferably from 200 to 2000.

(Synthesis of thiol compounds)

The thiol compounds used in the present invention may be synthesized by esterification between a mercapto group-containing carboxylic acid represented by Formula (2) and an alcohol.
The alcohols may be polyfunctional alcohols, whereby polyfunctional thiol compounds are obtained by the esterification.
Examples of the mercapto group-containing carboxylic acid represented by Formula (2) include 2-mercapto-2-phenylacetic acid, 3-mercapto-3-phenylpropionic acid and 4-mercapto-4-phenylbutanoic acid.
Examples of the polyfunctional alcohols include alkylene glycols (having a C2-10 optionally branched alkylene group), diethylene glycol, glycerin, diglycerin, dipropylene glycol, trimethylolpropane, pentaerythritol, dipentaerythritol, cyclohexanediol, cyclohexanedimethanol, norbornenedimethanol, norbornanedimethanol, polycarbonate diol, polysilicones having a hydroxyl group at both ends, and aromatic ring-containing polyols.
The alkylene glycols include ethylene glycol, trimethylene glycol, 1,2-propylene glycol, 1,2-butanediol, 1,3-butanediol, 2,3-butanediol, tetramethylene glycol, 1,5-pentanediol and 1,6-hexanediol.
The aromatic ring-containing polyols include bisphenol A, hydrogenated bisphenol A, bisphenol A dihydroxyethyl ether, 4,4'-(9-fluorenylidene)diphenol and 4,4'-(9-fluorenylidene)bis(2-phenoxyethanol).
Preferred polyfunctional alcohols include alkylene glycols (having a C2-10 optionally branched alkylene group), diethylene glycol, dipropylene glycol, glycerin, diglycerin, trimethylolpropane, pentaerythritol, dipentaerythritol, cyclohexanediol, cyclohexanedimethanol, norbornenedimethanol, bisphenol A, hydrogenated bisphenol A and 4,4'-(9-fluorenylidene)bis(2-phenoxyethanol).
The thiol compounds of the present invention may be produced by any methods without limitation. For example, the thiol compounds may be obtained by the esterification of the mercapto group-containing carboxylic acid of above Formula (2) and the alcohol according to a known method to produce an ester.
For example, objective thiol compounds may be obtained by the following process.
The mercapto group-containing carboxylic acid of above Formula (2) may be obtained as follows. Thiourea is added to an aqueous mineral acid solution and the mixture is heated with stirring. To the aqueous solution, a carboxylic acid compound in which a phenyl group is bonded to an unsaturated double bond is added and the mixture is heated with stirring, giving a thiuronium salt. The thiuronium salt is then hydrolyzed in an aqueous alkaline solution such as sodium hydroxide solution to give the mercapto group-containing carboxylic acid.
The mineral acids used herein are not particularly limited. Examples thereof include sulfuric acid, nitric acid and hydrochloric acid, with hydrochloric acid being preferable. Examples of the alkalis include inorganic alkalis such as sodium hydroxide, potassium hydroxide and sodium carbonate, and organic base compounds such as ammonia, diethylamine and triethylamine. Of these, the inorganic alkalis are preferable and sodium hydroxide is more preferable. The heating with stirring is preferably performed at 80 to 140°C, and more preferably 90 to 120°C. Examples of the carboxylic acid compounds in which a phenyl group is bonded to an unsaturated double bond include cinnamic acid and 4-phenyl-3-butenoic acid but are not limited thereto.
The mercapto group-containing carboxylic acid of Formula (2) synthesized as described above and the polyfunctional alcohol are dissolved in a solvent capable of forming an azeotropic mixture with water to allow for azeotropic dehydration. An acid catalyst is then added, and the mixture is heated under reflux while performing azeotropic dehydration to remove water formed by the esterification. The objective thiol compounds may be thus obtained.
The solvents are preferably capable of forming an azeotropic mixture with water to allow for azeotropic dehydration. Examples include aromatic solvents such as benzene, toluene, xylene, mesitylene, pentamethylbenzene and anisole, and ether solvents such as tetrahydrofuran and tetrahydropyran. Examples of the acid catalysts include mineral acids such as sulfuric acid, and organic acids such as methanesulfonic acid, p-toluenesulfonic acid and naphlenesulfonic

acid. The acid catalyst is preferably added at 0.5 to 5 wt%, and more preferably 1.0 to 3 wt% relative to the mercapto group-containing carboxylic acid.

The mercapto group-containing carboxylic acid is preferably added in an amount such that the number of moles of the carboxylic acid groups is 1.0 to 1.5 times, and more preferably 1.1 to 1.3 times the number of moles of the alcohol groups.

The esterification conditions are not particularly limited and may be appropriately selected from known reaction conditions.

(Curable compositions)

The curable compositions according to the present invention contain the thiol compound and a radically polymerizable compound. Examples of the radically polymerizable compounds include compounds having an ethylenically unsaturated double bond.

The compounds having an ethylenically unsaturated double bond used in the present invention are compounds that are cured by radical polymerization (or crosslinking) and addition reaction. Examples of such compounds include allyl alcohol derivatives, ethylenically unsaturated aromatic compounds, (meth)acrylic acid/polyhydric alcohol esters and (meth)acrylates such as urethane (meth) acrylate. These compounds may be used singly, or two or more kinds may be used in combination.

Examples of the ethylenically unsaturated aromatic compounds include styrene, $\alpha$-methylstyrene, o-methylstyrene, m-methylstyrene, p-methylstyrene, p-tert-butylstyrene, diisopropenylbenzene, o-chlorostyrene, m-chlorostyrene, p-chlorostyrene, 1,1-diphenylethylene, p-methoxystyrene, N,N-dimethyl-p-aminostyrene, N,N-diethyl-p-aminostyrene, ethylenically unsaturated pyridine and ethylenically unsaturated imidazole. Examples of the (meth) acrylates include carboxyl group-containing compounds such as (meth) acrylic acid, crotonic acid, maleic acid, fumaric acid and itaconic acid; alkyl (meth)acrylates such as methyl (meth)acrylate, ethyl (meth)acrylate, propyl (meth)acrylate, isopropyl (meth) acrylate, butyl (meth)acrylate, isobutyl (meth)acrylate, tert-butyl (meth)acrylate, pentyl (meth)acrylate, amyl (meth)acrylate, isoamyl (meth)acrylate, hexyl (meth)acrylate, heptyl (meth)acrylate, octyl (meth)acrylate, isooctyl (meth)acrylate, 2-ethylhexyl (meth)acrylate, nonyl (meth)acrylate, decyl (meth)acrylate, isodecyl (meth)acrylate, undecyl (meth)acrylate, dodecyl (meth)acrylate, lauryl (meth)acrylate, stearyl (meth)acrylate and isostearyl (meth)acrylate; fluoroalkyl (meth) acrylates such as trifluoroethyl (meth)acrylate, tetrafluoropropyl (meth)acrylate, hexafluoroisopropyl (meth)acrylate, octafluoropentyl (meth)acrylate and heptadecafluorodecyl (meth)acrylate; hydroxyalkyl (meth)acrylates such as hydroxyethyl (meth)acrylate, hydroxypropyl (meth) acrylate and hydroxybutyl (meth) acrylate; phenoxyalkyl (meth) acrylates such as phenoxyethyl (meth)acrylate and 2-hydroxy-3-phenoxypropyl (meth)acrylate; alkoxyalkyl (meth)acrylates such as methoxyethyl (meth)acrylate, ethoxyethyl (meth)acrylate, propoxyethyl (meth)acrylate, butoxyethyl (meth)acrylate and methoxybutyl (meth)acrylate; polyethylene glycol (meth) acrylates such as polyethylene glycol mono(meth)acrylate, ethoxydiethylene glycol (meth)acrylate, methoxypolyethylene glycol (meth)acrylate, phenoxypolyethylene glycol (meth) acrylate and nonylphenoxypolyethylene glycol (meth)acrylate; polypropylene glycol (meth)acrylates such as polypropylene glycol mono (meth) acrylate, methoxypolypropylene glycol (meth)acrylate, ethoxypolypropylene glycol (meth) acrylate and nonylphenoxypolypropylene glycol (meth)acrylate; cycloalkyl (meth)acrylates such as cyclohexyl (meth) acrylate, 4-butylcyclohexyl (meth)acrylate, dicyclopentanyl (meth)acrylate, dicyclopentenyl (meth)acrylate, dicyclopentadienyl (meth)acrylate, bornyl (meth)acrylate, isobornyl (meth)acrylate and tricyclodecanyl (meth)acrylate; benzyl (meth) acrylate, tetrahydrofurfuryl (meth)acrylate, ethylene glycol di(meth)acrylate, diethylene glycol di(meth)acrylate, triethylene glycol di(meth)acrylate, polyethylene glycol di(meth)acrylate, propylene glycol di(meth)acrylate, dipropylene glycol di(meth)acrylate, tripropylene glycol di(meth)acrylate, polypropylene glycol di(meth)acrylate, neopentyl glycol di(meth) acrylate, 1,3-propanediol di(meth)acrylate, 1,4-butanediol di(meth)acrylate, 1,6-hexanediol di(meth)acrylate, hydroxypivalate neopentyl glycol di(meth)acrylate, bisphenol A di(meth)acrylate, trimethylolpropane tri(meth)acrylate, pentaerythritol tri(meth)acrylate and trimethylolpropanetrioxyethyl (meth)acrylate.

Examples of the urethane (meth)acrylates include compounds in which 2-(meth)acryloyloxyethyl isocyanate, 2,2-bis(acryloyloxymethyl)ethyl isocyanate, 1,1-bis(acryloyloxymethyl)methyl isocyanate or 4-(meth)acryloyloxyphenyl isocyanate is added to active hydrogen compounds such as alcohols. Specific examples include compounds in which the isocyanate compounds as described above are added to polyols such as ethylene glycol, 1,3-propanediol, 1,4-butanediol, 1,6-hexanediol, trimethylolpropane, pentaerythritol, norbornenedimethanol, norbornanedimethanol, tris(2-hydroxyethyl)isocyanurate, polycarbonate diol, polysilicones having a hydroxyl group at both ends, and bisphenol A ethoxylate. These compounds may be used singly, or two or more kinds may be used in combination.

The compounds having an ethylenically unsaturated double bond are not limited to the above compounds as long as compounds have an ethylenically unsaturated group and are polymerizable. The compounds having an ethylenically unsaturated double bond may be high molecular-weight compounds containing an ethylenically unsaturated double bond in the molecule.

In the present invention, the thiol compound and the compound having an ethylenically unsaturated double

bond are preferably mixed such that the molar ratio of the mercapto groups in the thiol compound and the ethylenically unsaturated double bonds is in the range of 1:99 to 50:50, and particularly preferably 5:95 to 20:80.

In the present invention, polymerization initiators such as photopolymerization initiators and thermal polymerization initiators may be used together with the thiol compounds. The photopolymerization initiators induce polymerization and addition reaction by being irradiated with active energy rays such as UV rays, visible rays and electron beams, and thereby provide cured products. Specific examples of the photopolymerization initiators include 1-hydroxycyclohexyl phenyl ketone, 2,2'-dimethoxy-2-phenylacetophenone, xanthone, fluorene, fluorenone, benzaldehyde, anthraquinone, triphenylamine, carbazole, 3-methylacetophenone, 4-chlorobenzophenone, 4,4'-dimethoxybenzophenone, 4,4'-diaminobenzophenone, 4,4'-bis(N,N-diethylamino)benzophenone, Michler's ketone, benzoylpropyl ether, benzoin ethyl ether, benzyldimethyl ketal, 1-(4-isopropylphenyl)-2-hydroxy-2-methylpropan-1-one, 2-hydroxy-2-methyl-1-phenylpropan-1-one, thioxanthone, diethylthioxanthone, 2-isopropylthioxanthone, 2-chlorothioxanthone, 2-methyl-1-[4-(methylthio)phenyl]-2-morpholinopropan-1-one, 2,4,6-trimethylbenzoyldiphenylphosphine oxide, 2-benzyl-2-dimethylamino-1-(4-morpholinophenyl)butan-1-one, 1-[4-(2-hydroxyethoxy)-phenyl]-2-hydroxy-2-methylpropan-1-o ne and 2,2'-bis(2-chlorophenyl)-4,4',5,5'-tetraphenyl-1,2'-biimida zole.

Further, commercially available products may be used, with examples including IRGACURE 184, 651, 500 and 907, CG 1369, CG 24-61, DAROCUR 1116 and 1173 (manufactured by Ciba Specialty Chemicals Inc.), LUCIRIN LR 8728, TPO (manufactured by BASF), and UBECRYL (manufactured by UCB).

The polymerization initiators may be used singly, or two or more kinds may be used in combination.

The polymerization may also be induced by heat to give cured products. That is, thermal polymerization initiators may be used in the curable compositions. In some cases, the addition reaction may be induced in the absence of thermal polymerization initiators.

Examples of the thermal polymerization initiators include azo compounds such as azo-bis-diphenyl methane, 2,2'-azobisisobutyronitrile and dimethyl-2,2'-azo-bis(2-methylpropionate); organic peroxides such as diacyl peroxides, ketone peroxides, hydroperoxides, dialkyl peroxides and peroxy esters; and persulfates. These compounds may be used singly, or two or more kinds may be used in combination. Specific examples of the organic peroxides include benzoyl peroxide, 3,5,5-trimethylhexanoyl peroxide, lauroyl peroxide, stearoyl peroxide, octanoyl peroxide, di-n-propyl peroxydicarbonate, diisopropyl peroxydicarbonate, bis(4-t-butylcyclohexyl) peroxydicarbonate, di-2-ethoxyethyl peroxydicarbonate, di-2-ethylhexyl peroxydicarbonate, di-2-methoxybutyl peroxydicarbonate, di(3-methyl-3-methoxybutyl) peroxydicarbonate, 1,1,3,3-tetramethylbutyl peroxyneodecanoate, 1-cyclohexyl-1-methylethyl peroxyneodecanoate, t-hexyl peroxyneodecanoate, t-butyl peroxyneodecanoate, t-hexyl peroxypivalate, t-butyl peroxypivalate, 1,1,3,3-tetramethylbutyl peroxy-2-ethylhexanoate, 1-cyclohexyl-1-methylethyl peroxy-2-ethylhexanoate and t-hexyl peroxy-2-ethylhexanoate.

The amount of the polymerization initiators used is not particularly limited, but is preferably in the range of 0.1 to 20 parts by weight, and more preferably 0.5 to 10 parts by weight based on 100 parts by weight of the compounds having an ethylenically unsaturated double bond. If the amount of the polymerization initiators used is less than 0.1 parts by weight, the polymerization rate may be low or the polymerization may be inhibited by oxygen or the like. If the amount of the polymerization initiators used exceeds 20 parts by weight, termination reaction is dominant in the polymerization and the adherence strength or transparency may be adversely affected.

The thiol compounds may generally account for 10 to 90% by weight of the polymerization initiator composition.

The curable compositions of the present invention may contain sensitizers, adhesion improvers such as silane coupling agents and acidic phosphates, antioxidants, dyes, fillers, pigments, thixotropic agents, plasticizers, surfactants, lubricants and antistatic agents as required.

The curable compositions of the present invention achieve inhibited hydrogen abstraction reaction in the mercapto groups and facilitated addition reaction to the ethylenically unsaturated double bonds, by containing the secondary or higher thiol compounds which have two or more mercapto groups and in which the carbon atom at $\alpha$-position relative to the mercapto group has an aryl group, and the compounds having an ethylenically unsaturated double bond.

The curable compositions may be blended and prepared for example as follows. The thiol compounds according to the present invention, compounds having an ethylenically unsaturated double bond, and polymerization initiators are mixed together with a mixing device such as a mixer, a ball mill or a three roll mill, or are dissolved by addition of solvents as diluting agents, at room temperature or elevated temperatures.

The thiol compounds and compounds having an ethylenically unsaturated double bond are as described hereinabove. The solvents include esters such as ethyl acetate, butyl acetate and isopropyl acetate; ketones such as acetone, methyl ethyl ketone, methyl isobutyl ketone and cyclohexanone; cyclic ethers such as tetrahydrofuran and dioxane; amides such as N,N-dimethylformamide; aromatic hydrocarbons such as toluene; and halogenated hydrocarbons such as methylene chloride.

(Cured products)

The curable compositions may be cured by any methods without limitation. For example, the curable composition may be applied on a substrate to form a coated film and may be cured by irradiation, heating or a combination thereof.
The coated film thickness for testing properties is preferably in the range of 1 to 200 μm, but may be appropriately determined depending on use.
The application methods include use of die coaters, spin coaters, spray coaters, curtain coaters and roll coaters, as well as screen printing and dipping.
The radiations used herein are not particularly limited. Preferred examples include electron beams and rays in the range from ultraviolet to infrared. Examples of the light sources are ultrahigh pressure mercury lamps and metal halide light sources for UV rays, metal halide light sources and halogen light sources for visible rays, and halogen light sources for infrared rays. Laser sources and LEDs are also usable. Application of infrared rays also provides thermal curing. The irradiation dose may be determined appropriately depending on the type of light source and the coated film thickness.
The above curable compositions may be used in applications including resists (e.g., solder resists, etching resists, color filter resists, spacers), sealants (e.g., waterproof sealants), paints (e.g., antifouling paints, fluorine paints, aqueous paints), pressure-sensitive adhesives and bonding agents (e.g., adhesives, dicing tapes), printing plates (e.g., CTP plates, offset plates), print proofreading (e.g., color proofs), lenses (e.g., contact lenses, microlenses, optical waveguides), dental materials, surface treatments (e.g., optical fiber coating, disk coating) and battery materials (e.g., solid electrolytes).

## EXAMPLES

The present invention will be described below by presenting Examples and Comparative Examples without limiting the scope of the invention.

Synthetic Example 1:

Synthesis of 3-mercapto-3-phenylpropionic acid (hereinafter MPPA")

A 1-liter three-necked flask was charged with 25.3 g of thiourea (manufactured by Wako Pure Chemical Industries, Ltd.), 175 g of 36% hydrochloric acid (manufactured by JUNSEI CHEMICAL CO., LTD.) and 168 g of ion exchanged water. The mixture was heated at 120°C for 4 hours with stirring. Thereafter, 24.6 g of cinnamic acid (manufactured by Wako Pure Chemical Industries, Ltd.) was added, and the mixture was heated for 11 hours with stirring, resulting in precipitation of a light yellow solid. The reaction liquid was cooled to room temperature and was further cooled with ice water. Subsequently, 420 g of 28 wt% sodium hydroxide was added dropwise with stirring, and the temperature was increased to 90°C with stirring. During this process, the crystal was dissolved and a new crystal was precipitated. The reaction liquid was cooled to room temperature. While the liquid was further cooled with ice water, 210 g of 23% hydrochloric acid was added dropwise to neutralize the liquid. The liquid was then filtered through a Kiriyama funnel to give a crude crystal. The crude crystal was dissolved in 2 L of toluene (manufactured by JUNSEI CHEMICAL CO., LTD.) and washed with water. The toluene was distilled off and the distillate was dried to afford 17.5 g of MPPA (56.1% yield).

<$^1$H-NMR>

$^1$H-NMR chart of MPPA is shown in Fig. 1. The $^1$H-NMR measurement was carried out with use of JNM-AL400 (manufactured by JEOL Ltd.) in deuterated chloroform.
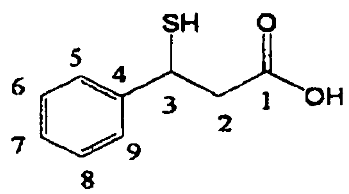

(16)

2.246-2.261 ppm: Hydrogen atom of the SH group

2.985-3.071 ppm: Hydrogen atoms of the methylene group at 2
4.425-4.476 ppm: Hydrogen atom of the methine group at 3
7.172-7.521 ppm: Hydrogen atoms of the phenyl group at 4 through 9

Synthetic Example 2

Synthesis of pentaerythritol tetrakis(3-mercapto-3-phenylpropionate) (hereinafter "PEMPP")

A 0.5-liter three-necked flask was charged with 2 g of pentaerythritol (manufactured by TOKYO CHEMICAL INDUSTRY CO., LTD.), 100 g of o-xylene (manufactured by TOKYO CHEMICAL INDUSTRY CO., LTD.), 13. 7 g of MPPA and 0.143 g of p-toluenesulfonic acid (manufactured by TOKYO CHEMICAL INDUSTRY CO., LTD.). A Dean-Stark apparatus and a condenser tube were attached. The mixture was heated at 155°C with stirring. After 8 hours after the initiation of the reaction, the reaction liquid was allowed to cool, washed with 100 ml of ion exchanged water, and neutralized with 200 ml of a 10% aqueous sodium hydrogen carbonate solution. The reaction liquid was further washed with ion exchanged water three times, and dehydrated and dried over anhydrous magnesium sulfate (manufactured by JUNSEI CHEMICAL CO., LTD.). Thereafter, the o-xylene was distilled off under reduced pressure and the distillate was vacuum dried to afford 10.0 g of PEMPP (84.0% yield).

<$^1$H-NMR>

$^1$H-NMR chart of PEMPP is shown in Fig. 2. The $^1$H-NMR measurement was carried out with use of JNM-AL400 (manufactured by JEOL Ltd.) in deuterated chloroform.
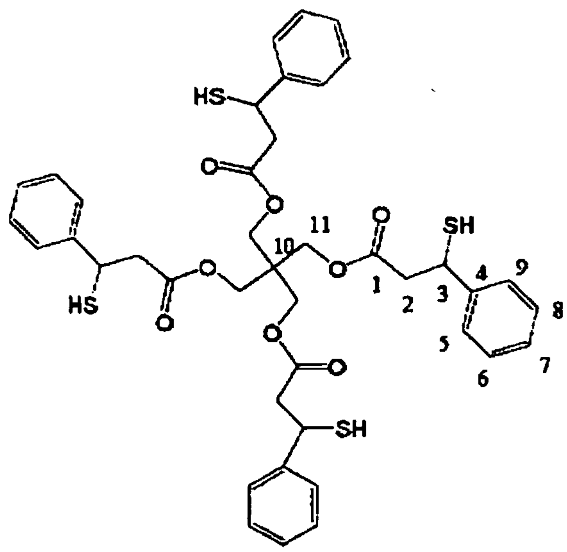

(6)

2.134-2.143 ppm: Hydrogen atoms of the SH groups
2.906-2.956 ppm: Hydrogen atoms of the methylene group at 2
3.961-3.967 ppm: Hydrogen atoms of the methylene group at 11
7.183-7.259 ppm: Hydrogen atoms of the phenyl group at 4 through 9

Synthetic Example 3

Synthesis of diglycerol tetra(3-mercapto-3-phenylpropionate) (hereinafter "DGMPP")

A 0.5-liter three-necked flask was charged with 3 g of diglycerol (manufactured by KANTO CHEMICAL CO., INC.), 100 g of o-xylene (manufactured by TOKYO CHEMICAL INDUSTRY CO., LTD.), 14. 7 g of MPPA and 0.4 g of p-toluenesulfonic acid (manufactured by TOKYO CHEMICAL INDUSTRY CO., LTD.). A Dean-Stark apparatus and a condenser tube were attached. The mixture was heated at 155°C with stirring.
After 15.5 hours after the initiation of the reaction, the reaction liquid was allowed to cool, washed with 100 ml

of ion exchanged water, and neutralized with 200 ml of a 10% aqueous sodium hydrogen carbonate solution. The reaction liquid was further washed with ion exchanged water three times, and dehydrated and dried over anhydrous magnesium sulfate (manufactured by JUNSEI CHEMICAL CO., LTD.). Thereafter, the o-xylene was distilled off under reduced pressure and the distillate was vacuum dried to afford 10.0 g of DGMPP (48.6% yield).

<$^1$H-NMR>

$^1$H-NMR chart of PEMPP is shown in Fig. 3. The $^1$H-NMR measurement was carried out with use of JNM-AL400 (manufactured by JEOL Ltd.) in deuterated chloroform.

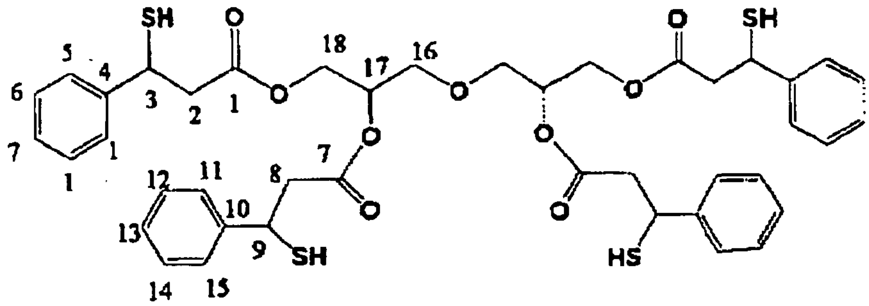

(7)

2.233 ppm: Hydrogen atoms of the SH groups
2.732-3.004 ppm: Hydrogen atoms of the methylene groups at 2 and 8
3.626-4.044 ppm: Hydrogen atoms of the methylene groups and the methine group at 16, 17 and 18
4.440 ppm: Hydrogen atom of the methylene group at 11

Synthetic Example 4:

1. Acrylic copolymer (AP) having carboxyl group and ethylenically unsaturated group

A four-necked flask equipped with a dropping funnel, a thermometer, a condenser tube, a stirrer and a nitrogen inlet tube was charged with 7.38 parts by mass of methacrylic acid (manufactured by MITSUBISHI RAYON CO., LTD.), 8. 63 parts by mass of p-methylstyrene (manufactured by Deltech Corp.), 0.05 parts by mass of mercaptoethanol (manufactured by Wako Pure Chemical Industries, Ltd.) and 23.45 parts by mass of PGME. The four-necked flask was purged with nitrogen at 90°C for 0.5 hour. Thereafter, a liquid mixture consisting of 23.45 parts by mass of PGME and 0.31 parts by mass of 2,2'-azobisisobutyronitrile (manufactured by Wako Pure Chemical Industries, Ltd., abbreviated to "AIBN") was added dropwise over a period of 1 hour. The resultant mixture was heated at 90°C with stirring for 3 hours. Subsequently, a liquid mixture consisting of 2. 61 parts by mass of cyclohexanone (manufactured by Wako Pure Chemical Industries, Ltd.) and 0.10 parts by mass of AIBN was added dropwise. The resultant mixture was heated at 90°C with stirring for 1.5 hours and further at 100°C with stirring for 1.0 hour, thereby giving an acrylic copolymer having a carboxyl group.

The acrylic copolymer having a carboxyl group obtained above was placed in a four-necked flask equipped with a thermometer, a condenser tube, a stirrer and an air inlet tube. To the flask, 2.30 parts by mass of triphenylphosphine (manufactured by TOKYO CHEMICAL INDUSTRY CO., LTD.), 0.16 parts by mass of hydroquinone (manufactured by TOKYO CHEMICAL INDUSTRY CO., LTD.), 21.82 parts by mass of glycidyl methacrylate (manufactured by MITSUBISHI RAYON CO., LTD.) and 16.45 parts by mass of 4-hydroxybutyl acrylate glycidyl ether (Nippon Kasei Chemical Co., Ltd.) were added. The flask was purged with air, and the mixture was heated at 100°C with stirring for 12 hours, and then allowed to cool. The resultant solution contained 35% by mass of an acrylic copolymer (AP) having a carboxyl group and an ethylenically unsaturated group (solvent: PGMEA). The weight average molecular weight of the AP (measured by GPC in terms of polystyrene standards) was 23,000.

Synthetic Example 5:

Synthesis of epoxy acrylate having carboxyl group (EA)

A reactor was charged with 185 parts by mass of EPIKOTE 1004 (bisphenol A epoxy resin, manufactured by Japan Epoxy Resins Co., Ltd., epoxy equivalent: 925), 14.4 parts by mass of acrylic acid, 0.20 parts by mass of hydroquinone and 197 parts by mass of diethylene glycol monoethyl ether acetate (abbreviated to "DGEA", manufactured by DAICEL CHEMICAL INDUSTRIES, LTD.). The mixture was heated to 95°C and uniformly dissolved. Thereafter, 2.0 parts by mass of triphenylphosphine was added, and the mixture was heated to 100°C. Reaction was carried out for approximately 30 hours to give a product having an acid value of 0.5 mg KOH/g. To the reaction product, 96.0 parts by mass of tetrahydrophthalic anhydride (manufactured by New Japan Chemical Co., Ltd.) was added. The resultant mixture was heated to 90°C and reacted for approximately 6 hours. IR confirmed the absence of absorption peaks assigned to the acid anhydride. The solution contained 60% by mass of epoxy acrylate resin (EA) having a solid acid value of 119 mg KOH/g (solvent: diethylene glycol monoethyl ether acetate).

Evaluation of photopolymerizable compositions:

[Reagents]

<Compounds (monomers) having ethylenically unsaturated group>

Dipentaerythritol hexaacrylate (DPHA): manufactured by DAICEL UCB Co., Ltd.
EO-modified bisphenol A diacrylate (BP4EA): manufactured by KYOEISHA CHEMICAL CO., LTD.

<Photopolymerization initiators>

1) EMK ((4,4'-bis(N,N-diethylamino)benzophenone):
manufactured by HODOGAYA CHEMICAL CO., LTD.
2) IRGACURE 907: manufactured by Ciba Specialty Chemicals Inc.
3) PEMB (pentaerythritol tetrakis(3-mercaptobutyrate)):
manufactured by Showa Denko K.K.

<Pigments>

1) Carbon black Special Black 350: manufactured by DEGUSSA
2) Titanium black 13 MC: manufactured by Mitsubishi Materials Corporation

<Others>

1) PMA (propylene glycol monoethyl ether acetate) : manufactured by TOKYO CHEMICAL INDUSTRY CO., LTD.
2) Cyclohexanone: manufactured by Wako Pure Chemical Industries, Ltd.
3) AJISPER PB822: dispersing agent manufactured by Ajinomoto Fine-Techno Co., Inc.

[Preparation of photopolymerizable compositions]

<Example 1>

4.44 Parts by mass of dispersing agent AJISPER PB822 was dissolved in a solvent mixture containing 212 parts by mass of PMA. Further, 9.5 parts by mass of EA (solid content: 5.7 parts by mass) was admixed therewith. 22.0 Parts by mass of carbon black Special Black 350 and 22.0 parts by mass of titanium black 13 MC were added as black pigments. The mixture was treated with a paint conditioner (manufactured by ASADA IRON WORKS, CO., LTD.) for 3 hours to give a fluid dispersion.

The fluid dispersion was combined with 93.5 parts by mass of the acrylic copolymer (AP) having a carboxyl group and an ethylenically unsaturated group (solid content: 32.7 parts by weight), 4.43 parts by mass of DPHA, 4.43 parts by mass of BP4EA and 103 parts by mass of PMA. Further, 0.5 parts by mass of EMK, 5 parts by mass of IRGACURE 907 and 1.0 parts by mass of PEMPP as photopolymerization initiators were added and dissolved in the mixture.

The thus-obtained composition was filtered through a 0.8 μm filter (Kiriyama filter paper for GFP) to give a photopolymerizable composition according to the present invention.

The photopolymerizable composition was evaluated for sensitivity by the method described later. The results are set forth in Table 1.

<Examples 2 to 5>

In Examples 2 to 5, photopolymerizable compositions were prepared in the same manner as in Example 1 except that the photopolymerization initiators were used as shown in Table 1. The photopolymerizable compositions were evaluated for sensitivity in the same manner as in Example 1. The results are set forth in Table 1.

<Examples 6 and 7>

In Examples 6 and 7, photopolymerizable compositions were prepared in the same manner as in Example 1 except that the photopolymerization initiators were used as shown in Table 1. The photopolymerizable compositions were heated at 60°C for 30 hours and then evaluated for sensitivity in the same manner as in Example 1. The results are set forth in Table 1.

<Comparative Examples 1 and 2>

Photopolymerizable compositions were prepared according to the formulation shown in Table 1 in the same manner as in Example 1 except that PEMB was used as a thiol compound. The photopolymerizable compositions were evaluated for sensitivity in the same manner as in Example 1. The results are set forth in Table 1.

<Comparative Example 3>

A photopolymerizable composition was prepared in the same manner as in Example 1 except that PEMB was used as a thiol compound. The photopolymerizable composition was heated at 60°C for 30 hours and then evaluated for sensitivity in the same manner as in Example 1. The results are set forth in Table 1.

[Evaluation of sensitivity]

The obtained photopolymerizable composition was spin coated on a glass substrate (100 x 100 mm) such that the dry thickness would be about 1.5 μm, and was dried at room temperature for 2 minutes and at 90°C for 3 minutes. The thickness of the dried film was precisely measured with a film thickness meter (SURFCOM 130A manufactured by TOKYO SEIMITSU CO., LTD.). The film was then exposed to light through a quartz photomask with an exposure apparatus equipped with an ultrahigh pressure mercury lamp ("Multilight ML-251 A/B" manufactured by USHIO INC.) while the dose was changed, whereby the photopolymerizable composition was cured. The dose was measured with an accumulated UV meter (trade name "UIT-150", receptor "UVD-S365", manufactured by USHIO INC.).

The exposed film was alkali developed for a predetermined time with a 0.1% aqueous sodium carbonate solution (25°C). The developing time was 1.5 times the time (tD) in which the film prior to the exposure was completely dissolved by the alkali development. The time tD was determined in repeated experiments in which the film was dissolved for various lengths of alkali developing time and the degree of dissolution was observed to determine the time tD in which the film was completely dissolved. After the alkali development, the film was washed with water and the glass substrate was dried by air spraying. The thickness of the residual film (resist) was measured and the residual film rate was calculated as follows:

Residual film rate (%) = 100 x (thickness after alkali development)/(thickness before alkali development)

According to the above equation, the residual film rate at a dose of 100 mJ/$cm^2$ was measured, and the sensitivity was compared among the compositions.

The results of Examples 1-7 and Comparative Examples 1-3 in Table 1, in particular comparison between Examples 2 and 5 with Comparative Example 2 and comparison between Examples 6 and 7 with Comparative Example 3, show that the substitution of the phenyl group to the carbon atom bonded to the mercapto group increases thermal stability although sensitivity (residual film rate) is slightly reduced.

[Table 1]

| Chemical composition of photopolymerizable compositions and residual film rate | | | | | | | | | | | | |
|---|---|---|---|---|---|---|---|---|---|---|---|---|
| | | Ex. 1 | Ex. 2 | Ex. 3 | Ex. 4 | Ex. 5 | Ex. 5 | Ex. 6 | Ex. 7 | Comp. Ex. 1 | Comp. Ex. 2 | Comp. Ex. 3 |
| Photopolymerization initiators (parts by mass) | | | | | | | | | | | | |
| Sensitizer | EMK | 0.5 | 0.5 | 0.5 | 0.5 | 0.5 | 0.5 | 0.5 | 0.5 | 0.5 | 0.5 | 0.5 |
| Light-induced radical generator | IRGACURE 907 | 5.0 | 5.0 | 5.0 | 5.0 | 5.0 | 5.0 | 5.0 | 5.0 | 5.0 | 5.0 | 5.0 |
| Thiol compounds | PEMPP (*1) | 1.0 | 2.0 | 3.0 | | | | 2.0 | | | | |
| | DGMPP (*2) | | | | 1.0 | 2.0 | 3.0 | | 2.0 | | | |
| | PEMB (*3) | | | | | | | | | 0.0 | 2.0 | 2.0 |
| Results | | | | | | | | | | | | |
| Residual film rate (%) (*4) | | 92 | 95 | 97 | 93 | 95 | 98 | 93 | 94 | 80 | 98 | 90 |

(*1) PEMPP: pentaerythritol tetrakis(3-mercapto-3-phenylpropionate)
(*2) DGMPP: diglycerol tetra(3-mercapto-3-phenylpropionate)
(*3) PEMB: pentaerythritol tetrakis(3-mercaptobutyrate)
(*4) Residual film rate (%): at 100 mJ/$cm^2$ dose

## Claims

1. A polymerization accelerator comprising a thiol compound, the thiol compound having two or more groups represented by Formula (1) below:

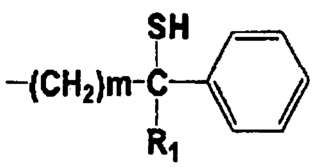

(1)

wherein $R_1$ is a hydrogen atom or a C1-10 alkyl group, and m is an integer of 0, 1 or 2.

2. The polymerization accelerator according to claim 1, wherein the thiol compound is an ester compound between a mercapto group-containing carboxylic acid represented by Formula (2) below and a polyfunctional alcohol:

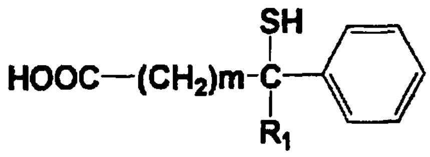

(2)

wherein $R_1$ and m are the same as $R_1$ and m in Formula (1) described in claim 1.

**3.** The polymerization accelerator according to claim 2, wherein the polyfunctional alcohol is a compound selected from the group consisting of alkylene glycols (having a C2-10 optionally branched alkylene group), diethylene glycol, dipropylene glycol, glycerin, diglycerin, trimethylolpropane, pentaerythritol, dipentaerythritol, cyclohexanediol, cyclohexanedimethanol, norbornenedimethanol, bisphenol A, hydrogenated bisphenol A and 4,4'-(9-fluorenylidene) bis(2-phenoxyethanol).

**4.** The polymerization accelerator according to claim 2, wherein the thiol compound is represented by Formula (3) below:

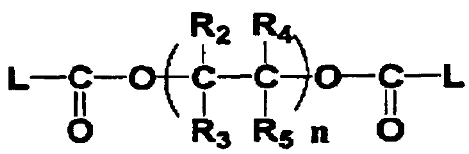

(3)

wherein $R_2$ to $R_5$ are each independently a hydrogen atom or a C1-10 alkyl group, n is an integer of 1 to 3, and L is a group represented by Formula (1) according to claim 1.

**5.** The polymerization accelerator according to claim 2, wherein the thiol compound is represented by Formula (4) below:

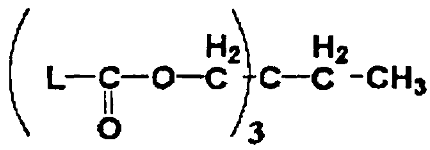

(4)

wherein L is a group represented by Formula (1) according to claim 1.

**6.** The polymerization accelerator according to claim 2, wherein the thiol compound is represented by Formula (5) below:

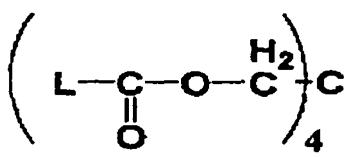

(5)

wherein L is a group represented by Formula (1) according to claim 1.

**7.** The polymerization accelerator according to claim 2, wherein the thiol compound is represented by Formula (6) below:

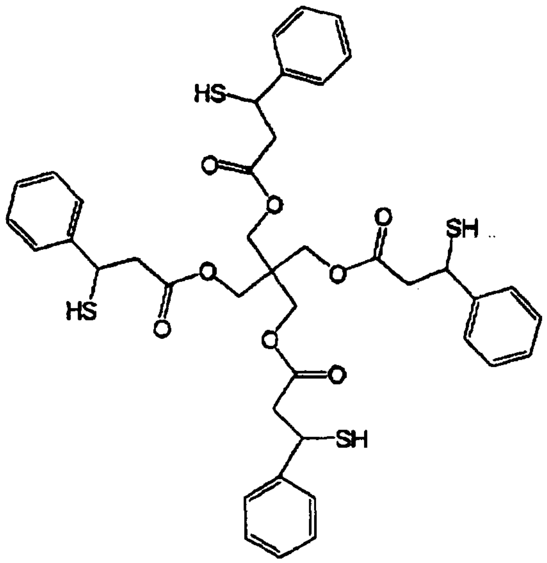

(6)

8. The polymerization accelerator according to claim 2, wherein the thiol compound is represented by Formula (7) below:

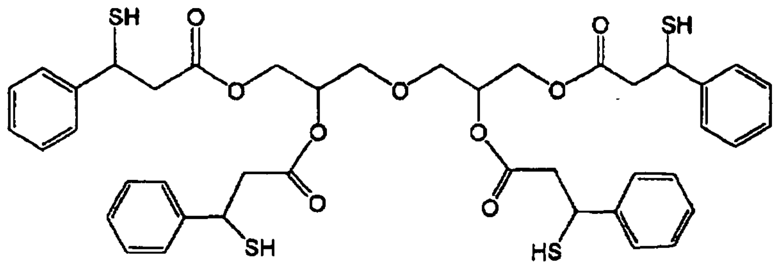

(7)

9. A curable composition comprising the thiol compound described in any one of claims 1 to 8 and a radically polymerizable compound.

10. A curable composition comprising the thiol compound described in any one of claims 1 to 8 and a compound having an ethylenically unsaturated double bond.

11. A cured product obtained from the curable composition described in claim 9 or 10.

12. A process for producing a thiol compound having two or more groups represented by Formula (1) according to claim 1, the process comprising dissolving a mercapto group-containing carboxylic acid represented by Formula (2) according to claim 2 and at least one compound selected from the polyfunctional alcohols according to claim 3 in a solvent capable of forming an azeotropic mixture with water to allow for azeotropic dehydration; adding an acid catalyst; heating the mixture under reflux; and performing azeotropic dehydration to remove water formed by the esterification.

[Fig. 1]

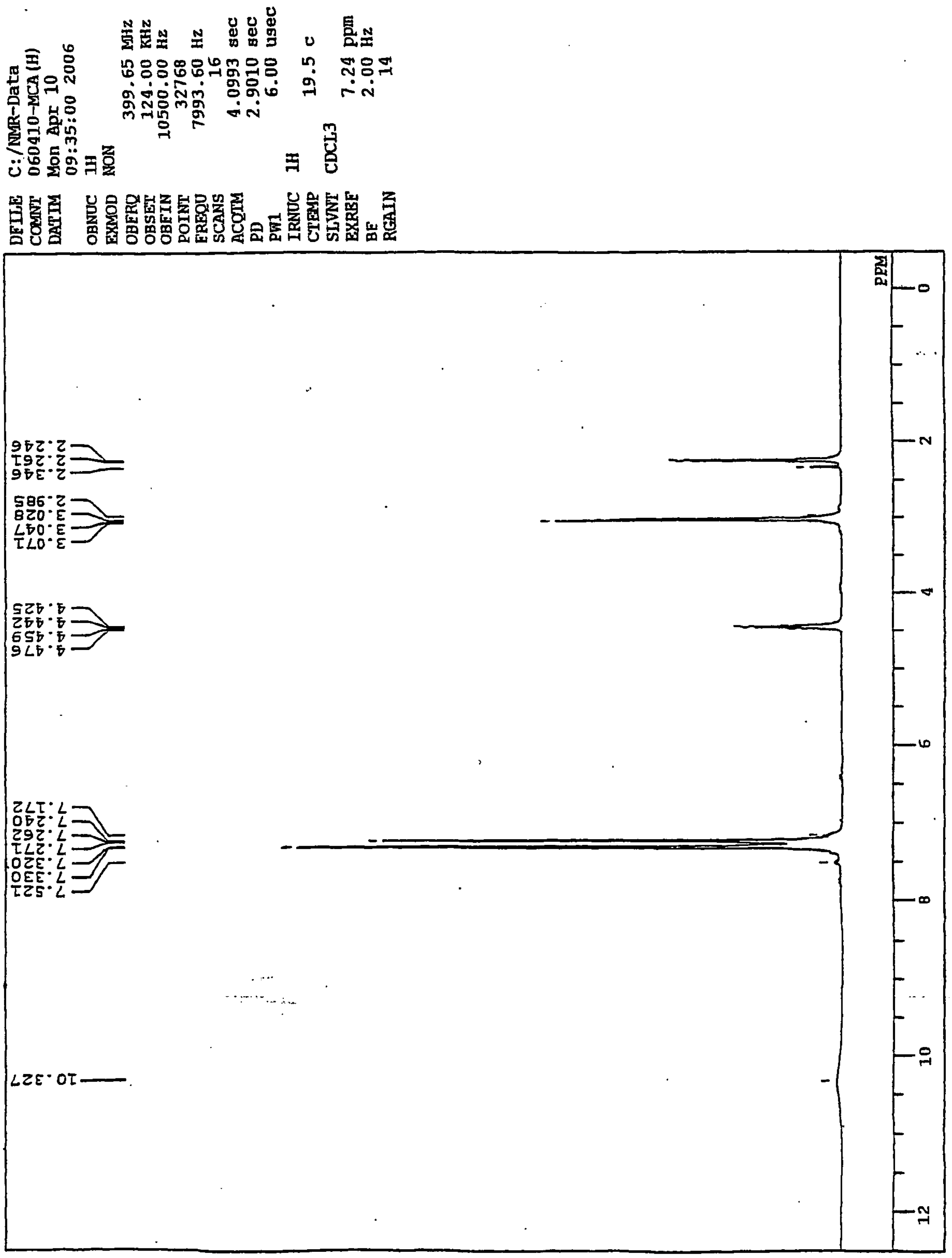
DFILE C:/NMR-Data
COMNT 060410-MCA(H)
DATIM Mon Apr 10
09:35:00 2006
OBNUC 1H
EXMOD NON
OBFRQ 399.65 MHz
OBSET 124.00 KHz
OBFIN 10500.00 Hz
POINT 32768
FREQU 7993.60 Hz
SCANS 16
ACQTM 4.0993 sec
PD 2.9010 sec
PW1 6.00 usec
IRNUC 1H
CTEMP 19.5 c
SLVNT CDCL3
EXREF 7.24 ppm
BF 2.00 Hz
RGAIN 14
2.246
2.261
2.346
2.985
3.028
3.047
3.071
4.425
4.442
4.459
4.476
7.172
7.240
7.262
7.271
7.320
7.330
7.521
10.327
PPM
0
2
4
6
8
10
12

[Fig. 2]

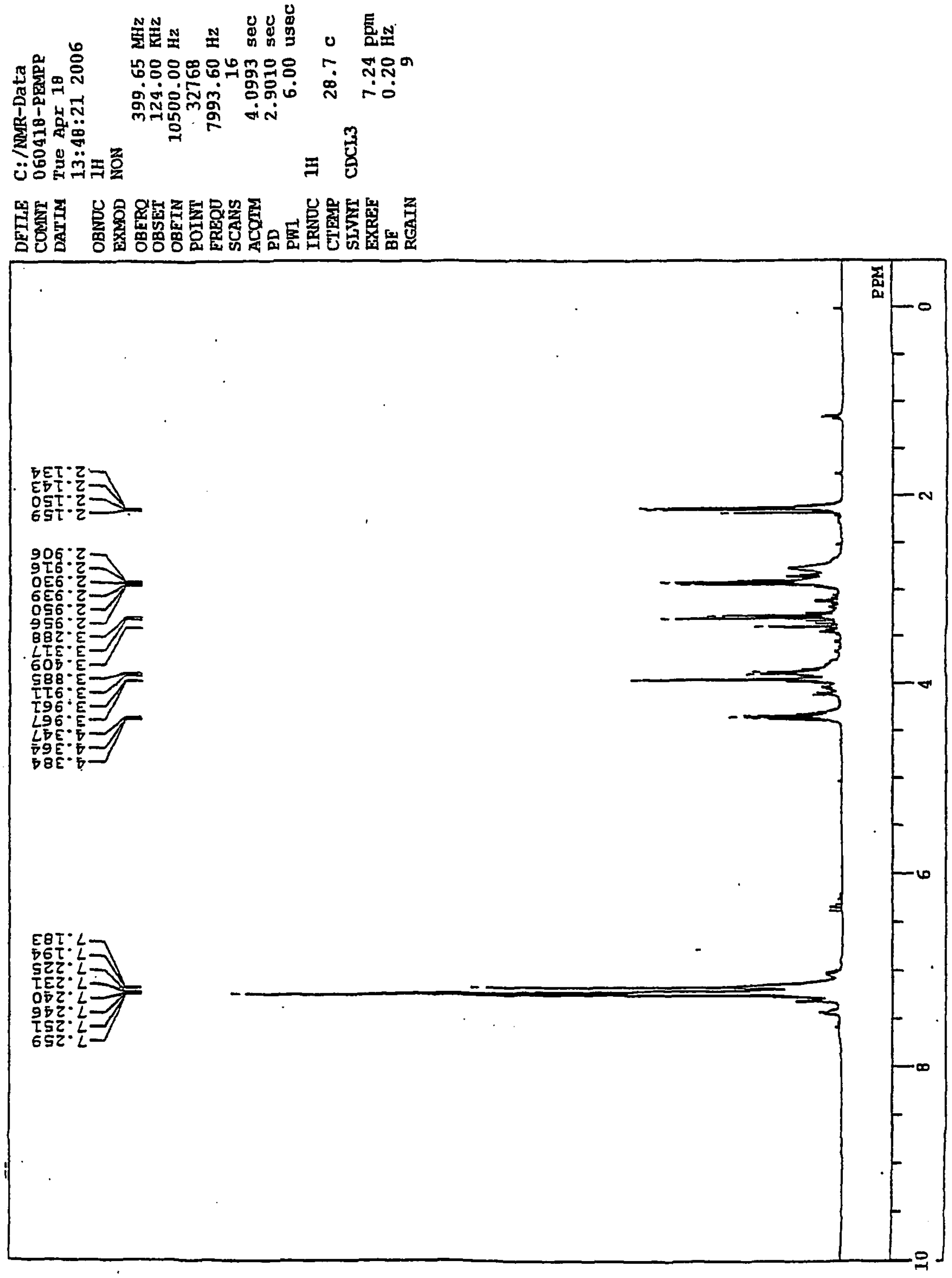
DFILE C:/NMR-Data
COMNT 060418-PEMPP
DATIM Tue Apr 18 13:48:21 2006
OBNUC 1H
EXMOD NON
OBFRQ 399.65 MHz
OBSET 124.00 KHz
OBFIN 10500.00 Hz
POINT 32768
FREQU 7993.60 Hz
SCANS 16
ACQTM 4.0993 sec
PD 2.9010 sec
PW1 6.00 usec
IRNUC 1H
CTEMP 28.7 c
SLVNT CDCL3
EXREF 7.24 ppm
BF 0.20 Hz
RGAIN 9
2.134
2.143
2.150
2.159
2.906
2.916
2.930
2.939
2.950
2.956
3.288
3.317
3.409
3.885
3.911
3.961
3.967
4.347
4.364
4.384
7.183
7.194
7.225
7.231
7.240
7.246
7.251
7.259
PPM
0
2
4
6
8
10

[Fig. 3]

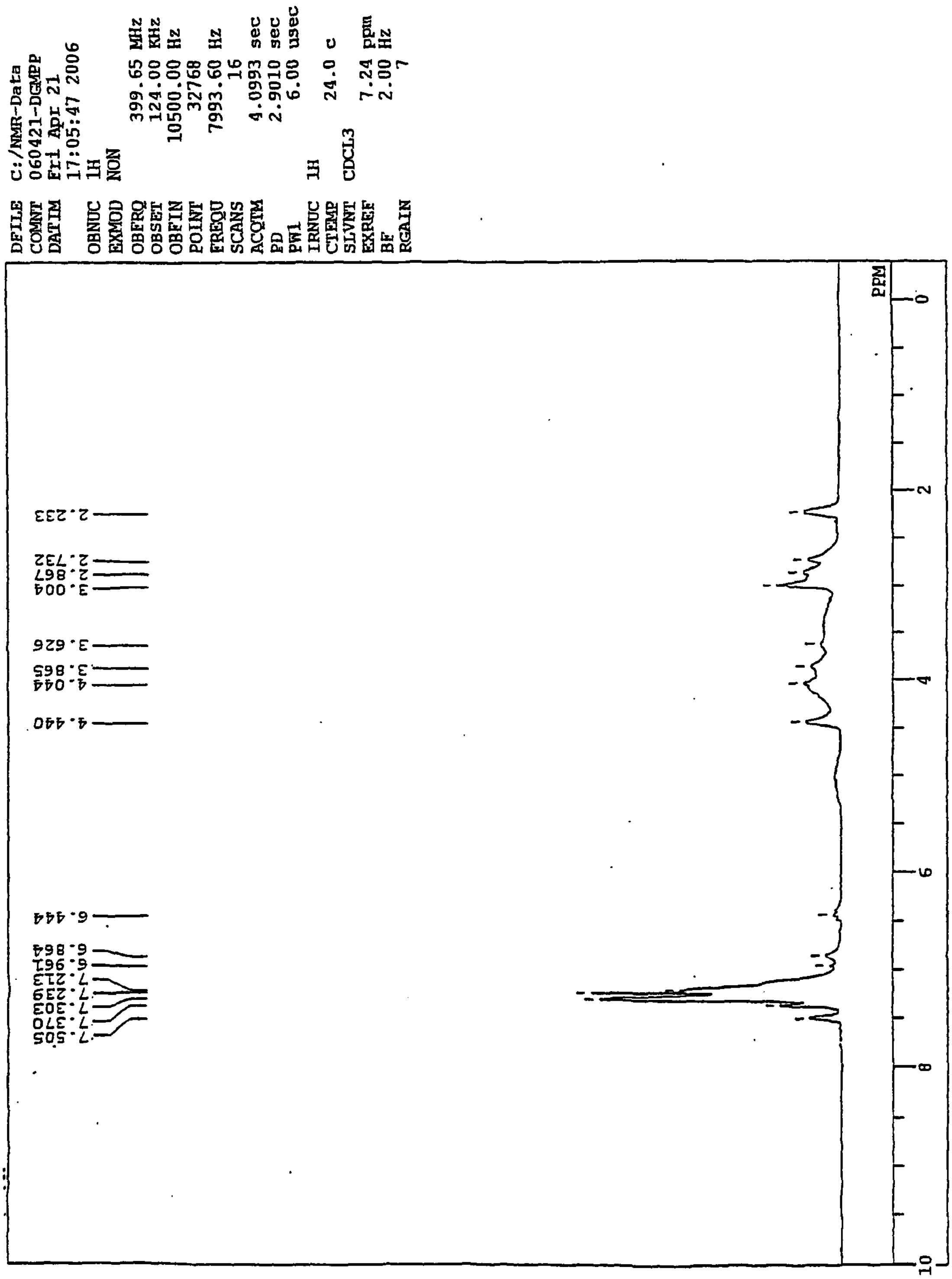
DFILE C:/NMR-Data
COMNT 060421-DGMPP
DATIM Fri Apr 21 17:05:47 2006
OBNUC 1H
EXMOD NON
OBFRQ 399.65 MHz
OBSET 124.00 KHz
OBFIN 10500.00 Hz
POINT 32768
FREQU 7993.60 Hz
SCANS 16
ACQTM 4.0993 sec
PD 2.9010 sec
PW1 6.00 usec
IRNUC 1H
CTEMP 24.0 c
SLVNT CDCL3
EXREF 7.24 ppm
BF 2.00 Hz
RGAIN 7
2.233
2.732
2.867
3.004
3.626
3.865
4.044
4.440
6.444
6.864
6.961
PPM
0
2
4
6
8
10

**INTERNATIONAL SEARCH REPORT**

International application No.
PCT/JP2007/061885

A. CLASSIFICATION OF SUBJECT MATTER
*C08F2/38*(2006.01)i, *C07C319/12*(2006.01)i, *C07C323/56*(2006.01)i, *C07B61/00*(2006.01)n

According to International Patent Classification (IPC) or to both national classification and IPC

B. FIELDS SEARCHED

Minimum documentation searched (classification system followed by classification symbols)
C08F2/38, C07C319/12, C07C323/56, C07B61/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CA(STN)

C. DOCUMENTS CONSIDERED TO BE RELEVANT

| Category* | Citation of document, with indication, where appropriate, of the relevant passages | Relevant to claim No. |
|---|---|---|
| A | JP 2005-76018 A (Denki Kagaku Kogyo Kabushiki Kaisha), 24 March, 2005 (24.03.05), Claims; Par. Nos. [0009] to [0011] (Family: none) | 1-12 |
| A | JP 2004-264435 A (Showa Denko Kabushiki Kaisha), 24 September, 2004 (24.09.04), Claims (Family: none) | 1-12 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 31 July, 2007 (31.07.07) | 07 August, 2007 (07.08.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2003226718 A
- JP 2003277505 A
- JP 2001026608 A
- JP 2004149755 A